# EUROPEAN PATENT SPECIFICATION

(11) **EP 3 547 834 B1**
(45) Date of publication and mention of the grant of the patent: **04.08.2021**
(21) Application number: 17809259.9
(22) Date of filing: 01.12.2017
(51) Int. Cl.: A01N 1/02

(54) **SUSTAINED RELEASE COMPOSITION**
ZUSAMMENSETZUNGEN MIT VERZÖGERTER FREISETZUNG
COMPOSITIONS À LIBÉRATION PROLONGÉE

(30) Priority: 05.12.2016 EP 16202112
(43) Date of publication of application: 09.10.2019
(73) Proprietor: Spermvital AS, 2317 Hamar (NO)
(72) Inventor: KOMMISRUD, Elisabeth, N-2335 Stange (NO); ALM-KRISTIANSEN, Anne, Hege, N-2324 Vang på Hedmarken (NO); KLINKENBERG, Geir, N-7072 Heimdal (NO)
(74) Representative: Onsagers AS
(86) International application number: PCT/EP2017/081128
(87) International publication number: WO 2018/104160

(56) References cited:
- EP-A1- 2 526 768
- WO-A1-2013/076232
- WO-A1-2015/181496
- WO-A2-01/40370
- WO-A2-2008/004890
- .: "Alginate 3d Cell Culture Kit", , 16 December 2012 (2012-12-16), pages 1-10, XP055336843, Tokyo, Japan Retrieved from the Internet: URL:http://search.cosmobio.co.jp/cosmo_sea rch_p/search_gate2/docs/CSR_/ABCKIT.201302 19.pdf [retrieved on 2017-01-19]
- Michael L O'Connor: "HEAT DETECTION AND TIMING OF INSEMINATION FOR CATTLE", , 1 January 1993 (1993-01-01), pages 1-20, XP055337310, Pennsylvania State University Retrieved from the Internet: URL:http://extension.psu.edu/animals/dairy /health/reproduction/insemination/ec402/ex tension_publication_file [retrieved on 2017-01-20]
- James M Flink ET AL: "A novel method for immobilization of yeast cells in alginate gels of various shapes by internal liberation of Ca-ions", Biotechnology Letters, 1 October 1985 (1985-10-01), pages 765-768, XP55400047, DOI: 10.1007/BF01032293 Retrieved from the Internet: URL:https://rd.springer.com/content/pdf/10 .1007/BF01032293.pdf [retrieved on 2017-08-21]
- Brian Amsden ET AL: "Diffusion Characteristics of Calcium Alginate Gels", BIOTECHNOLOGY AND BIOENGINEERING, 5 December 1999 (1999-12-05), pages 605-610, XP55400048, Retrieved from the Internet: URL:http://onlinelibrary.wiley.com/store/1 0.1002/(SICI)1097-0290(19991205)65:5<605:: AID-BIT14>3.0.CO;2-C/asset/14_ftp.pdf?v=1& t=j6mdjgfw&s=7ae9b5ce0b48a2ea311bcdd3ad2b1 8890e18ece9 [retrieved on 2017-08-21]

## Description

### Field of the invention

The present invention relates to sustained release compositions, and in particular a sustained release composition comprising non-human spermatozoa embedded in a matrix, such as a polymeric matrix. By having at least two sections with distinct dissolution rate, the matrix will ensure that non-human spermatozoa are released for a prolonged period of time and thus making the time of insemination relative to ovulation less critical. Further, the present invention also relates to a method for preparing the sustained release composition and container comprising the sustained release composition.

### Background of the invention

Artificial insemination (AI) is a technique where spermatozoa are placed into an animal's uterus or cervix by artificial means rather than by natural copulation. The technique has been used since the 1940s and is now widely utilized as a method of mating and in breeding of animals to propagate desirable characteristics, particularly in the case of farm animals such as cattle, swine, sheep, poultry and horses, but also in case of pets such as pedigree dogs, aquatic animals and endangered species.

An overview over the development of modern AI and the challenges of the breeders as regards the use of artificial insemination and preservation of spermatozoa is disclosed in R. H. Foote (2002), American Society of Animal Science and in "Reproduction in farm animals", edited by B. Hafez, E.S.E. Hafez. - 7th ed., Philadelphia, Lippincott Williams & Wilkins, 2000. - XIII, ISBN 0-683-30577-8 (ib.).

Spermatozoa are typically collected, extended and then preserved e.g. by cryopreservation. The use of cryopreservation techniques presupposes that the spermatozoa from the specific species of animal tolerate such treatment without resulting in too much deterioration of the spermatozoa quality, viability and fertilization capacity. The spermatozoa are then typically transported to the female's location either cryopreserved or freshly stored, whichever is suitable. It is vital that the spermatozoa are maintained viable until the time of insemination and for a sufficient period of time inside the female animal after insemination until the egg cell(s) reach the location of fertilization.

There has been a lot of focus and research for preservation methods aiming at providing storage methods and means which ensure that the spermatozoa maintain the fertilizing capacity for a longer period of time after collection and till the point of insemination. However, a spermatozoa preservation system which ensures that the spermatozoa maintain the fertilizing capacity for a longer period of time also after insemination would be beneficial for the breeding industry in general as the time of insemination relative to ovulation would be less critical.

WO2008/004890 discloses a preservation system where spermatozoa are embedded in alginate. The preservation system is said to provide benefits i.a. by giving the spermatozoa fertilizing capacity for a longer period of time after insemination.

EP2526768 discloses a composition comprising spermatozoa and a matrix polymer in an extender composition. The purpose of the alginate is to increase the density of the extender and thereby avoid settling out of the spermatozoa.

The aim of WO2015/181496 is to reduce the loss of spermatozoa, and in particular reduce the number of spermatozoa that are absorbed into the plug of an insemination straw during filling. This object is achieved by impregnating the plug with a fast-acting cross linker (i.e. not an inactive crosslinker), such as BaCh, which quickly will react with sodium alginate during filling to form a gel. The gel will ensure that the spermatozoa are not absorbed into the plug during filling.

Even though good preservation systems already have been disclosed, there is still a need for improved preservation systems that provides higher viability both during storage, before and after insemination. A system where the breeder is less dependent on meeting the most preferable insemination point in time in respect of ovulation would provide more flexibility and result in an increase in successful impregnation.

### Summary of the invention

The present inventors have solved this need by providing a sustained release composition, and in particular a sustained release composition comprising non-human spermatozoa embedded in a matrix, such as a polymeric matrix. By having at least two sections with distinct dissolution rate, the matrix ensures that non-human spermatozoa with high fertilizing capacity is released for a prolonged period of time and thus making the time of insemination relative to ovulation less critical.

The present invention provides in a **first aspect** a sustained release composition comprising a material to be released embedded in a matrix, the matrix comprising an ionically crosslinked polymer and having at least a first and a second section with distinct mechanical strength; wherein the ionically crosslinked polymer is ionically crosslinked alginate, the alginate having more guluronic acid residues than mannuronic acid residues.

According to particular embodiments, the material to be released is non-human spermatozoa.

Where spermatozoa are mentioned in the present disclosure, this always refers to non-human spermatozoa.

According to particular embodiments, the ionically crosslinked alginate is divalent cation crosslinked alginate, the alginate having more guluronic acid residues than mannuronic acid residues.

According to particular embodiments, the first section has a mechanical strength that is at least 10% higher, such as at least 20% higher, than the mechanical strength of the second section.

According to particular embodiments, the second section has a mechanical strength in the range 10 to 190 g/mm and the first section has a mechanical strength that is at least 10% higher, such as at least 20% higher, than the mechanical strength of the second section.

According to particular embodiments, the second section has a mechanical strength in the range 10 to 190 g/mm and the first section has a mechanical strength in the range 10 to 190 g/mm, with the proviso that the first and a second sections have distinct mechanical strength.

According to particular embodiments, the mechanical strength of the first section : the mechanical strength of the second section ratio is at least 1.1, such as at least 1.2.

According to particular embodiments,
- the material to be released is non-human spermatozoa; and
- the ionically crosslinked polymer is divalent cation crosslinked alginate, the alginate having more guluronic acid residues than mannuronic acid residues.

The present invention provides in a **second aspect** a method for preparing the sustained release composition of the first aspect of the present invention, the method comprising the following steps:
- mixing the material to be released with a composition comprising ionically crosslinkable alginate thereby forming a pre-mixture;
- bringing the pre-mixture into contact with an inactive crosslinker and an activator composition; and
- allowing the ions and the ionically crosslinkable alginate to form ionically crosslinked alginate and thereby obtain the sustained release composition of the first aspect of the present invention;
wherein
the ionically crosslinkable alginate has more guluronic acid residues than mannuronic acid residues;
the inactive crosslinker is capable of releasing ions upon activation, the ions being suitable for crosslinking the ionically crosslinkable alginate; and the activator composition comprising one or more compounds capable of activating the inactive crosslinker.

According to particular embodiments, the material to be released is non-human spermatozoa.

According to particular embodiments, the inactive crosslinker releases ions, such as divalent ions, when exposed to a pH ≤ 8, such as a pH ≤ 7.5 or a pH ≤ 7 or a pH ≤ 6. According to particular embodiments, the inactive crosslinker is a divalent cation carbonate, such as CaCO₃, BaCO₃ or any mixture thereof.

According to particular embodiments, the inactive crosslinker and/or the activator composition are/is in solid form. When they are in solid form they cannot react with each other.

According to particular embodiments, the activator composition comprises glucono-δ-lactone (GDL).

According to particular embodiments, the activator composition either directly activates the inactive crosslinker or initiates a series of events resulting in the activation of the inactive crosslinker.

According to particular embodiments, the pre-mixture is brought into contact with the inactive crosslinker and then subsequently the activator composition is added.

According to particular embodiments, the pre-mixture is brought into contact with the activator composition and then subsequently the inactive crosslinker is added.

According to particular embodiments, the pre-mixture is brought into contact with the activator composition and the inactive crosslinker, the activator composition and the inactive crosslinker being simultaneously added to the pre-mixture.

According to particular embodiments, the pre-mixture further comprises a hydrolase; and the activator composition comprises a substrate being hydrolysable by the hydrolase.

According to particular embodiments, the activator composition comprises a hydrolase; and the pre-mixture further comprises a substrate being hydrolysable by the hydrolase.

According to particular embodiments, the inactive crosslinker is in solid form.

According to particular embodiments,
- the material to be released is non-human spermatozoa;
- the inactive crosslinker is a divalent cation carbonate, such as CaCO₃, BaCO₃ or any mixture thereof;
- the inactive crosslinker is in solid form; and
- the activator composition comprises a hydrolase and the pre-mixture further comprises a substrate being hydrolysable by the hydrolase; or
the premixture further comprises a hydrolase and the activator composition comprises a substrate being hydrolysable by the hydrolase; or
the activator composition comprises GDL .

The present invention provides in a **third aspect the** sustained release composition according to the first aspect of the present invention, the sustained release composition being obtainable by the method according to the second aspect of the present invention.

The present invention provides in a **fourth aspect** a container, such as an insemination straw or tube, comprising the sustained release composition according to the first or third aspect of the present invention; wherein the material to be released is non-human spermatozoa.

### Brief description of drawings

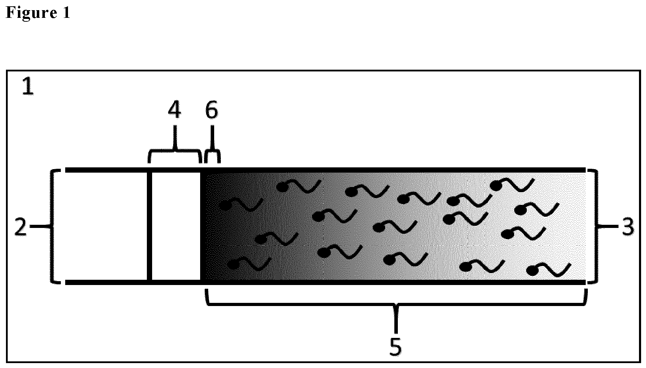

**Figure 1** illustrates an insemination straw (1) comprising a tube extending between a first end (2) and a second end (3) and comprising a gas-permeable, liquid-tight plug (4), said plug being arranged in the tube in the vicinity of the first end of same and extending between a first end turned towards the first end of the tube (2) and a second end turned towards the second end of the tube (3). A mixture of CaCO₃ and GDL in solid form (6) is positioned in the vicinity of the end of the gas-permeable, liquid-tight plug (4) facing the second end of the tube (3). A mixture comprising spermatozoa and alginate is introduced into the straw (5) thereby forming an ionically crosslinked polymer with embedded spermatozoa. Dark grey color indicates high degree of crosslinking, grey color indicates medium degree of crosslinking and light grey color indicates low degree of crosslinking.

### Detailed description of the invention

Unless specifically defined herein, all technical and scientific terms used have the same meaning as commonly understood by a skilled artisan in the fields of polymer engineering, biochemistry, molecular biology and animal breeding.

Where a numerical limit or range is stated herein, the endpoints are included. Also, all values and sub ranges within a numerical limit or range are specifically included as if explicitly written out.

It has previously been shown that embedding spermatozoa in an alginate matrix provides benefits by giving the spermatozoa fertilizing capacity for a longer period of time after insemination. It is assumed that the embedding results in immobilization of the spermatozoa within the alginate matrix, and that the restricted movement results in reduced energy consumption by the spermatozoa which is beneficial as regards shelf life and fertilization capacity.

Based on the above, it seems reasonable to assume that the degree of immobilization would influence the energy consumption by the spermatozoa, and thereby their shelf life and fertilization capacity. Since the degree of immobilization will typically vary depending on the characteristics of the matrix, such as e.g. mechanical strength, such characteristics would be expected to have a direct effect on shelf life and fertilization capacity.

A crosslinked polymer, such as an ionically crosslinked polymer, with low degree of crosslinking will have reduced mechanical strength compared to an ionically crosslinked polymer with high degree of crosslinking. Such a matrix would have fewer constraints within the matrix and allow for higher degree of movement, which in view of the above would be expected to be unfavorable as regards shelf life and fertilization capacity.

The present invention is based on the surprising finding that the mechanical strength of the matrix may be significantly reduced without negatively affecting shelf life and fertilization capacity of the embedded spermatozoa. Since the mechanical strength is directly linked to the dissolution rate of the matrix, this finding makes it possible to adjust the dissolution profile of the matrix without negatively affecting shelf life and fertilization capacity of the embedded spermatozoa.

By being able to adjust the dissolution profile without negatively affecting shelf life and fertilization capacity, a system may be designed where spermatozoa are continuously released from the polymeric matrix for a prolonged period after insemination. Since shelf life and fertilization capacity is maintained while being embedded within the polymeric matrix, the continuous release ensures that there are high quality spermatozoa available for fertilization for a prolonged period after insemination thus making the time of insemination relative to ovulation less critical.

It is to be understood that even though the present invention was designed for use in the breeding of animals, and in particular artificial insemination, the inventive concept may also find other fields of utilization. Examples of other fields of utilization may be administration of therapeutic agents, such as therapeutic active compounds, diagnostic agents or biological materials, such as cells and in particular stem cells.

Thus, a **first aspect** of the present invention relates to a sustained release composition comprising a material to be released embedded in a matrix, such as a polymeric matrix, the matrix comprising an ionically crosslinked polymer and having at least a first and a second section with distinct mechanical strength; wherein the ionically crosslinked polymer is ionically crosslinked alginate, the alginate having more guluronic acid residues than mannuronic acid residues.

The sustained release composition described herein is designed to release a material at a predetermined rate in order to ensure continuous supply of the material for a specific period of time and at the same time ensure that non-released material remains preserved within the matrix until the point of release.

The term "sustained release" is taken to encompass controlled release, prolonged release, timed release, retarded release, extended release and delayed release.

In one embodiment the material to be released is released for a period of at least 2 hours after insemination, such as at least 4 hours after insemination, at least 8 hours after insemination, at least 16 hours after insemination, at least 32 hours after insemination or at least 144 hours after insemination. In another embodiment, the material to be released is released for a period in the range 2-144 hours after insemination, such as 4-120 hours after insemination, 8-96 hours after insemination, 16-72 hours after insemination, 24-96 hours after insemination, 24-120 hours after insemination or 24-144 hours after insemination.

In one embodiment according to the present invention, the material to be released is selected from the group consisting of biological material, such as cells and in particular stem cells, therapeutic agents, diagnostic agents or any mixture thereof. In one preferred embodiment according to the present invention, the material to be released is non-human spermatozoa.

The term "non-human spermatozoa" as used herein includes non-human spermatozoa as such and also non-human spermatozoa contained in seminal fluid. i.e., non-human semen may be used directly when forming the sustained release composition according to the present invention. However, non-human spermatozoa isolated from the seminal fluid, optionally contained in other suitable storage solutions, may also be used to form the sustained release composition according to the present invention.

The material to be released is embedded in a matrix. The term "embedded" as used herein means that the material to be released is prevented from having its natural possibility of movement. The degree of immobilization will vary depending on the characteristics of the matrix, such as e.g. mechanical strength. However, it is to be understood that the material to be released, e.g. the non-human spermatozoa, embedded in the matrix according to the present invention is prevented from having its natural possibility of movement that the material to be released otherwise would have if it was stored in liquid, such as in a liquid core of a capsule.

The term "matrix" as used herein refers to the matrix into which the material to be released is embedded. The matrix provides for reduced possibility of movement, and the degree of immobilization will typically vary depending on the characteristics of the matrix, such as e.g. mechanical strength. However, not only the degree of immobilization but also the dissolution rate of the matrix will typically vary depending on the characteristics of the matrix. A matrix with high degree of crosslinking will typically have higher mechanical strength and therefore lower dissolution rate than a matrix with low degree of crosslinking. Thus, by combining two matrixes with distinct mechanical strength, the combined matrix will have two sections with distinct mechanical strength. Since high mechanical strength is associated with low dissolution rate and low mechanical strength is associated with high dissolution rate, the two sections will have distinct dissolution rates.

The matrix according to the present invention has at least a first and a second section with distinct mechanical strength. If the matrix has two sections with distinct mechanical strength, one of the sections will have lower mechanical strength than the other. If the matrix has three or more sections, at least two of these will have distinct mechanical strength.

In one embodiment according to the present invention, the matrix has at least three sections, such as at least four sections, at least five sections, at least six sections, at least seven sections, at least eight sections, at least nine sections or at least ten sections with distinct mechanical strength. In another embodiment according to the present invention, the matrix has a plurality of sections with distinct mechanical strength.

According to other embodiments, the matrix has at least a first, a second and a third section with distinct mechanical strength. More preferably, the matrix has at least a first, a second, a third and a fourth section with distinct mechanical strength. Even more preferably, the matrix has at least a first, a second, a third, a fourth, a fifth, a sixth, a seventh, an eighth, a ninth and a tenth section with distinct mechanical strength.

According to other embodiments, the number of sections with distinct mechanical strength is >10, more preferably >50, even more preferably >100 and most preferably > 200.

If the difference in mechanical strength between the sections is small, the matrix will in practice gradually dissolve section by section. However, if the difference in mechanical strength between the sections is high, the matrix will typically follow a time-staggered dissolution profile.

The sustained release composition according to the present invention may be divided into a predetermined number of sections. Each of the sections representing part of the sustained release composition. The sections may be of equal or different volumetric size.

In one embodiment, the first and second sections with distinct mechanical strength are similar or equal in volumetric size. The first and second sections with distinct mechanical strength may be positioned adjacent to each other in the sustained release composition or may not be positioned adjacent to each other in the sustained release composition.

In one embodiment according to the present invention, the matrix has at least three sections with distinct mechanical strength, such as at least five sections, at least seven sections, at least nine sections, at least eleven sections, at least thirteen sections, at least fifteen sections, at least seventeen sections or at least twenty sections.

The sustained release composition according to the present invention may in principle take any three-dimensional shape such as sphere, torus, cylinder, cone, cube, cuboid, triangular pyramid, square pyramid, triangular prism or any combination thereof.

In one embodiment according to the present invention, the matrix has the shape of a cylinder, cone, cube, cuboid, triangular pyramid, square pyramid, or a triangular prism. In one embodiment, the matrix has an increasing mechanical strength along the length of the three-dimensional shape. In another embodiment, the matrix has an increasing mechanical strength along the width of the three-dimensional shape.

In another embodiment according to the present invention, the matrix has the shape of a sphere, torus or cube. In one embodiment, the matrix has an increasing mechanical strength along an axis extending from the circumference towards the center of the shape. In another embodiment, the matrix has a decreasing mechanical strength along an axis extending from the circumference towards the center of the shape.

The term "dissolution rate" quantifies the speed of the dissolution process. Since the dissolution rate may vary depending on parameters such as temperature and the chemical nature of the solvent, the dissolution rate as used herein is measured according to the methods described in example 4.

In one embodiment according to the present invention, the dissolution rate as used herein is measured according to the methods described in example 4, in particular the in-vitro method for assessment of dissolution rate in the absence of calcium. Two sections are considered to have distinct dissolution rates if the dissolution rate of the first section is significantly different from the dissolution rate of the second section under identical conditions as measured by the methods described in example 4, in particular the in-vitro method for assessment of dissolution rate in the absence of calcium.

In an alternative embodiment according to the present invention, the dissolution rate as used herein is measured according to the methods described in example 4, in particular the in-vitro method for assessment of dissolution rate in the presence of calcium. Two sections are considered to have distinct dissolution rates if the dissolution rate of the first section is significantly different from the dissolution rate of the second section under identical conditions as measured by the methods described in example 4, in particular the in-vitro method for assessment of dissolution rate in the presence of calcium.

The dissolution rate of a section of the matrix reflects the time needed to completely dissolve the matrix within that section. The matrix is considered to be completely dissolved when there are no parts left having a size of more than 1 mm. In case of a sphere, the size of 1 mm refers to the longest diameter of the sphere. Similarly, in case of a cylindrical particle, the size of 1 mm refers to the longest length of the cylindrical particle. Similar applies to other geometrical shapes.

The term "mechanical strength" as used herein is measured according to the method described in example 5. Two sections are considered to have distinct mechanical strength if the mechanical strength of one of the sections is significantly different from the mechanical strength of the other section as measured by the method described in example 5. In one embodiment, two sections are considered to have distinct mechanical strength if the mechanical strength of the first section : the mechanical strength of the second section ratio is not equal to 1.

In one embodiment according to the present invention, the first section has a mechanical strength that is at least 5% higher, such as at least 10% higher, at least 15% higher, at least 20% higher, at least 25% higher, at least 30% higher, at least 35% higher, at least 40% higher, at least 45% higher, at least 50% higher, at least 60% higher, at least 70% higher, at least 80% higher, at least 90% higher, at least 100% higher, at least 200% higher or at least 400% higher than the mechanical strength of the second section. The mechanical strength is measured according to the method described in example 5. If one section has a mechanical strength of 20 g/mm and a second section has a mechanical strength of 40 g/mm, the mechanical strength of the latter section is 100% higher than the mechanical strength of the first mentioned section.

In another embodiment according to the present invention, the second section has a mechanical strength in the range 10 to 190 g/mm, such as e.g. 10 to 170 g/mm, 10 to 150 g/mm, 10 to 130 g/mm, 10 to 110 g/mm, 10 to 90 g/mm, 10 to 70 g/mm, 10 to 50 g/mm or 10 to 30 g/mm. In another embodiment according to the present invention, the second section has a mechanical strength in the range 30 to 190 g/mm, such as e.g. 50 to 190 g/mm, 70 to 190 g/mm, 90 to 190 g/mm, 110 to 190 g/mm, 130 to 190 g/mm, 150 to 190 g/mm or 170 to 190 g/mm. The mechanical strength of the section being measured by the method described in example 5.

In another embodiment according to the present invention, the second section has a mechanical strength in the range 10 to 190 g/mm, such as e.g. 10 to 170 g/mm, 10 to 150 g/mm, 10 to 130 g/mm, 10 to 110 g/mm, 10 to 90 g/mm, 10 to 70 g/mm, 10 to 50 g/mm or 10 to 30 g/mm and the first section has a mechanical strength in the range 10 to 190 g/mm, such as e.g. 10 to 170 g/mm, 10 to 150 g/mm, 10 to 130 g/mm, 10 to 110 g/mm, 10 to 90 g/mm, 10 to 70 g/mm, 10 to 50 g/mm or 10 to 30 g/mm; with the proviso that the mechanical strength of the first and second section is not identical, i.e. that the mechanical strength of the first and second section is different. Preferably, the first section has a mechanical strength that is at least 5% higher, such as at least 10% higher, at least 15% higher, at least 20% higher, at least 25% higher, at least 30% higher, at least 35% higher, at least 40% higher, at least 45% higher, at least 50% higher, at least 60% higher, at least 70% higher, at least 80% higher, at least 90% higher, at least 100% higher, at least 200% higher or at least 400% higher than the mechanical strength of the second section.

In another embodiment according to the present invention, the second section has a mechanical strength in the range 10 to 190 g/mm and the first section has a mechanical strength that is at least 5% higher, such as at least 10% higher, at least 15% higher, at least 20% higher, at least 25% higher, at least 30% higher, at least 35% higher, at least 40% higher, at least 45% higher, at least 50% higher, at least 60% higher, at least 70% higher, at least 80% higher, at least 90% higher, at least 100% higher, at least 200% higher or at least 400% higher than the mechanical strength of the second section. The mechanical strength being measured according to the method described in example 5.

In another embodiment according to the present invention, the mechanical strength of the second section is in the range 10 to 190 g/mm and the mechanical strength of the first section is higher than the mechanical strength of the second section. The mechanical strength of the sections being measured by the method described in example 5.

In another embodiment according to the present invention, the second section has a mechanical strength in the range 10 to 190 g/mm and the first section has a mechanical strength in the range 10 to 190 g/mm, with the proviso that the first and second sections have distinct mechanical strength. The mechanical strength of the sections being measured by the method described in example 5.

In another embodiment according to the present invention, the mechanical strength of the first section : the mechanical strength of the second section ratio is in the range 1.1 to 20, such as in the range 1.2 to 20, 1.3 to 20, 1.4 to 20, 1.5 to 20, 1.6 to 20, 1.7 to 20, 1.8 to 20, 2 to 20, 4 to 20 or 8 to 20. If a first section has a mechanical strength of 40 g/mm and a second section has a mechanical strength of 20 g/mm, the mechanical strength of the first section : the mechanical strength of the second section ratio is 2. It is to be understood that the first section has a higher mechanical strength than the second section.

The matrix according to the present invention comprises an ionically crosslinked polymer. The matrix may include further organic polymers, whether naturally occurring or synthetic, and whether homopolymers or copolymers. Preferably, the matrix is a biocompatible matrix, such as a biocompatible polymeric matrix, meaning that the matrix passes appropriate biological tests for short-term or long-term toxicity. In one embodiment according to the present invention, the matrix does not include further polymers, in particular further ionically crosslinked polymers.

The term "ionically crosslinkable polymers" refers to polymers whose linear or branched macromolecules may be linked to one another by way of ionic bonds to form three dimensional polymer networks. Once the ionically crosslinkable polymers are linked to one another by way of ionic bonds, the polymer is referred to as an ionically crosslinked polymer.

The ionically crosslinkable polymers may be anionic or cationic in nature and may include but are not limited to carboxylic, sulfate, and amine functionalized polymers such as polyacrylic acid, polymethacrylic acid, polyethylene amine, polysaccharides such as alginic acid, pectinic acid, carboxy methyl cellulose, hyaluronic acid, heparin, chitosan, carboxymethyl chitosan, carboxymethyl starch, carboxymethyl dextran, heparin sulfate, chondroitin sulfate, cationic guar, cationic starch, and their salts. Preferred ionically crosslinkable polymers are alginic acid, pectinic acid, carboxymethyl cellulose, hyaluronic acid, chitosan, and their salts. The most preferred ionically crosslinkable polymer is alginic acid and its salts, such as alginate and sodium alginate in particular.

The crosslinking ions are generally classified as anions or cations. Appropriate crosslinking ions include but are not limited to cations comprising an ion selected from the group consisting of calcium, magnesium, barium, strontium, boron, beryllium, aluminum, iron, copper, cobalt, lead, and silver ions, and anions selected from the group consisting of phosphate, citrate, borate, succinate, maleate, adipate and oxalate ions. More broadly, the anions are derived from polybasic organic or inorganic acids. Preferred crosslinking cations are calcium, barium, iron, and aluminum ions. The most preferred crosslinking cation is calcium.

In one embodiment according to the present invention, the crosslinking ion is a divalent ion, such as calcium or barium, and the ionically crosslinkable polymer is alginate, such as sodium alginate.

Alginate is an anionic copolymer of 1,4-linked-β-D-mannuronic acid and α-L-guluronic acid. Various forms of alginate are available commercially. Such forms are typically 60% 1,4-linked-β-D-mannuronic acid and 40% α-L-guluronic acid; or 30% 1,4-linked-β-D-mannuronic acid and 70% α-L- guluronic acid.

The ionically crosslinked polymer is alginate having more guluronic acid residues than mannuronic acid residues. In particular embodiments, the ionically crosslinked polymer is alginate being composed of >50% guluronic acid residues, such as >60% guluronic acid residues, >70% guluronic acid residues or >80% guluronic acid residues. The percentage being calculated based on the total number of residues in the alginate polymer. An ionically crosslinked polymer having 100 guluronic acid residues and 400 mannuronic acid residues is composed of 20% guluronic acid residues and 80% mannuronic acid residues.

Alginates are widely used e.g. in food industry as e.g. stabilizers and for viscosity control, in pharmaceutical and cosmetic industry as e.g. disintegrant. For the various purposes, both alginates being rich in guluronic acid or mannuronic acid, respectively, are available (Mancini et al., (1999), Journal of Food Engineering 39, 369 -378) and various methods for producing alginates being rich in guluronic acid are known, cf. WO 8603781, US 4,990,601, US 5,639,467. Ionically crosslinkable alginate having more guluronic acid residues than mannuronic acid residues is commercially available. One example being PROTANAL LF 10/60 available from FMC Biopolymer AS, Drammen, Norway which has a guluronic acid content of about 70%.

In one preferred embodiment according to the present invention, the sustained release composition of the present invention provides continuing release of the material to be released. The meaning of the term "continuing release" refers to a situation where more and more material is released as time passes by, i.e. that there is no point in time between start of release and completion where there is no release of material. In order to ensure that there is a continuing release of material, there should always be at least one section that is releasing material in the period from start of release until completion.

All sections of the sustained release composition are believed to start dissolving at the same time, but the dissolution rate will typically vary depending on the mechanical strength of each section. The section with the lowest mechanical strength will have the highest dissolution rate while the section with the highest mechanical strength will have the lowest dissolution rate provided that the sections have the same shape and volumetric size. If the density/concentration of the material to be released in each section is identical and all sections have the same shape and volumetric size, the sustained release composition may to a certain extent be expected to provide a decreasing rate of release from start to completion, i.e. that the rate of release is higher at start of release as compared to rate of release at completion. This effect may be accounted for e.g. by having a higher density of the material to be released in sections with high mechanical strength or by increasing the surface area of the sustained release composition in sections with high mechanical strength.

For illustrative purposes, reference is made to a sustained release composition having three sections of distinct mechanical strength, wherein the difference in mechanical strength between sections is high. Each section has the same shape and volumetric size, and the density/concentration of the material to be released in each section is identical. All sections are believed to start dissolving at the same time, but the dissolution rate between sections will vary depending on the mechanical strength of the composition of each section. The section with the lowest mechanical strength will have the highest dissolution rate. Since the density/concentration of the material to be released in each section is identical, the section with the lowest mechanical strength will release more material per time unit than the other sections. Thus, when the section with the lowest mechanical strength has been totally dissolved, it may be expected that the rate of release from the sustained release composition will decrease. When the next section is totally dissolved, it may be expected that the rate of release will drop even further.

As long as the sustained release composition releases sufficient amount of material per time unit to ensure that the desired result is achieved, the above mentioned effect will have no practical importance. However, if for some reason it should be desirable to ensure close to constant rate of release from start to completion, the above mentioned effect may be accounted for e.g. by ensuring i) that sections with higher mechanical strength have higher density/concentration of the material to be released than sections with lower mechanical strength; and/or ii) that sections with higher mechanical strength have larger surface area than sections with lower mechanical strength.

A section with high mechanical strength and high density/concentration of the material to be released may have the same rate of release as a section with low mechanical strength and low density/concentration of the material to be released. Similarly, a section with high mechanical strength and large surface area may have the same rate of release as a section with low mechanical strength and small surface area.

The term "rate of release" as referred to herein is intended to mean the amount of the "material to be released" that is released from the sustained release composition per time unit. The rate of release may be constant, fluctuate or vary from start of release until completion, i.e. until all sections of the sustained release composition have been completely dissolved.

In one embodiment according to the present invention, the rate of release is substantially constant from start of release until completion. In another embodiment according to the present invention, the rate of release is substantially the same in said first and second section, more preferably the rate of release is substantially the same in all sections. In another embodiment according to the present invention the rate of release fluctuate or varies from start of release until completion.

Independent of whether the sustained release composition provides a substantially constant rate of release, fluctuating rate of release or varying rate of release from start of release until completion; it is preferred that the sustained release composition provides continuing release of the material to be released over a period of at least 1 hour, such as at least 5 hours, at least 10 hours, at least 12 hours, at least 18 hours, at least 24 hours such as at least 48 hours.

In one embodiment according to the present invention, the ionically crosslinked alginate of the sustained release composition is not obtained by bringing a composition comprising alginate into contact with a multivalent, in particular a divalent (such as barium, calcium or magnesium) or a trivalent cation (such as aluminum or chromium) salt which has a solubility in water (25 °C, pH=7) higher than 1g/L, such as higher than 10g/L or higher than 100g/L. CaCh and BaCh have a solubility in water (25 °C, pH=7) around 811g/L and 358g/L respectively.

In a preferred embodiment according to the present invention, the ionically crosslinked alginate of the sustained release composition of the present invention is obtained by bringing a composition comprising alginate into contact with a multivalent and in particular a divalent (such as barium, calcium or magnesium) or trivalent cation (such as aluminum or chromium) salt which has a solubility in water (25 °C, pH=7) that is lower than 1g/L, such as a water solubility lower than 0.5g/L, lower than 0.05g/L or lower than 0.04g/L. CaCO₃ and BaCO₃ have a solubility in water (25 °C, pH=7) around 0.013g/L and 0.024g/L respectively.

In one embodiment according to the first aspect of the present invention, the ionically crosslinked alginate of the sustained release composition of the present invention is obtained by bringing a composition comprising alginate into contact with an inactive crosslinker as referred to below in respect of the second aspect of the present invention. It is to be understood that all passages referring to the inactive crosslinker of the second aspect of the present invention are also relevant in respect of the first aspect of the present invention.

In another embodiment according to the first aspect of the present invention, the ionically crosslinked alginate of the sustained release composition of the present invention is not obtained by bringing a composition comprising alginate into contact with a fast acting crosslinker, such as an easily dissolvable salt of barium, calcium or magnesium; or an easily dissolvable salt of a multivalent cation such as trivalent cations and in particular aluminium or chromium cations.

In another embodiment according to the first aspect of the present invention, the ionically crosslinked alginate of the sustained release composition of the present invention is obtained by bringing a composition comprising alginate into contact with a source of multivalent cations, in particular divalent cations such as barium, calcium or magnesium ions or trivalent cations such as aluminium or chromium ions. Said source of multivalent cations preferably being in a form ensuring sustained release of cations from the source of multivalent cations when brought in solution.

In another embodiment according to the first aspect of the present invention, the ionically crosslinked alginate of the sustained release composition of the present invention is obtained by bringing a composition comprising alginate into contact with a source of multivalent cations, in particular divalent cations such as barium, calcium or magnesium ions or trivalent cations such as aluminium or chromium ions.

In one preferred embodiment, the present invention relates to a sustained release composition comprising a material to be released embedded in a matrix, the matrix comprising an ionically crosslinked polymer and having at least a first and a second section with distinct mechanical strength; wherein
- the material to be released is non-human spermatozoa; and
- the ionically crosslinked polymer is divalent cation crosslinked alginate, such as Ca²⁺ crosslinked alginate, the alginate having more guluronic acid residues than mannuronic acid residues.

A **second aspect** of the present invention relates to a method for preparing the sustained release composition of the first aspect of the present invention, the method comprising the following steps:
- mixing the material to be released with a composition comprising ionically crosslinkable alginate thereby forming a pre-mixture;
- bringing the pre-mixture into contact with an inactive crosslinker and an activator composition; and
- allowing the ions and the ionically crosslinkable alginate to form ionically crosslinked alginate and thereby obtain the sustained release composition of the first aspect of the present invention;
wherein
the ionically crosslinkable alginate has more guluronic acid residues than mannuronic acid residues;
the inactive crosslinker is capable of releasing ions upon activation, the ions being suitable for crosslinking the ionically crosslinkable alginate; and
the activator composition comprising one or more compounds capable of activating the inactive crosslinker.

The first step of the method according to the second aspect of the present invention involves mixing the material to be released with a composition comprising an ionically crosslinkable polymer thereby forming a pre-mixture.

In one embodiment according to the present invention, the material to be released is selected from the group consisting of biological material, such as cells and in particular stem cells, therapeutic agents, diagnostic agents or any mixture thereof. In one preferred embodiment according to the present invention, the material to be released is non-human spermatozoa.

The term "ionically crosslinkable polymer" refers to a polymer whose linear or branched macromolecules may be linked to one another by way of ionic bonds to form three-dimensional polymer networks.

The ionically crosslinkable polymer may be anionic or cationic in nature and may include but are not limited to carboxylic, sulfate, and amine functionalized polymers such as polyacrylic acid, polymethacrylic acid, polyethylene amine, polysaccharides such as alginic acid, pectinic acid, carboxy methyl cellulose, hyaluronic acid, heparin, chitosan, carboxymethyl chitosan, carboxymethyl starch, carboxymethyl dextran, heparin sulfate, chondroitin sulfate, cationic guar, cationic starch, and their salts. Preferred ionically crosslinkable polymer is alginic acid, pectinic acid, carboxymethyl cellulose, hyaluronic acid, chitosan, and their salts. The most preferred ionically crosslinkable polymer is alginic acid and its salts, such as alginate and sodium alginate in particular.

In one embodiment according to the present invention, the ionically crosslinked polymer is alginate having more guluronic acid residues than mannuronic acid residues. In particular embodiments, the ionically crosslinked polymer is alginate being composed of >50% guluronic acid residues, such as >60% guluronic acid residues, >70% guluronic acid residues or >80% guluronic acid residues.

The second step of the method according to the second aspect of the present invention involves bringing the product of the first step into contact with an inactive crosslinker and an activator composition.

The term "inactive crosslinker" refers to a compound that requires activation by an activator composition in order to be able to release ions, the ions being suitable for crosslinking the ionically crosslinkable alginate. If the inactive crosslinker is not activated by an activator composition, the inactive crosslinker is not able to release ions suitable for crosslinking the ionically crosslinkable alginate. Said in other words, if the "inactive crosslinker" is mixed with a sodium alginate solution at neutral pH in the absence of an activator composition, ionically crosslinked alginate is not formed. However, if the "inactive crosslinker" is mixed with a sodium alginate solution in the presence of an activator composition, ionically crosslinked alginate will be formed. The inactive crosslinker is typically a compound which does not release ions when brought in contact with water at 25 °C, pH>8; the ions being suitable for crosslinking the ionically crosslinkable alginate. Thus, CaCl₂ and BaCl₂ are not inactive crosslinkers according to the definition provided herein, while CaCO₃ and BaCO₃ are examples of inactive crosslinkers.

In one embodiment according to the present invention, the inactive crosslinker is a divalent cation salt which has a solubility in water (25 °C, pH=7) that is lower than 1g/L, such as a water solubility lower than 0.5g/L, lower than 0.05g/L or lower than 0.04g/L. Examples of divalent cation salts fulfilling these requirements are divalent cation carbonates such as CaCO₃ and BaCO₃. Most preferably, the "inactive crosslinker" is CaCO₃.

In one embodiment according to the present invention, the inactive crosslinker is a compound that releases ions, such as cations and in particular divalent cations, in response to a decrease in pH. A divalent cation carbonate, such as CaCO₃ or BaCO₃, are examples of inactive crosslinkers that releases ions in response to a decrease in pH.

The term "activator composition" refers to a composition comprising one or more compounds capable of activating the inactive crosslinker. When the inactive crosslinker is activated, ions suitable for crosslinking the ionically crosslinkable alginate will be released from the activated crosslinker.

If the inactive crosslinker is CaCO₃, the activator composition may be any composition which is capable of interacting with CaCO₃ in such a way that free calcium ions are released from the carbonate salt.

One example of an activator composition which is able to activate CaCO₃ is a composition comprising GDL. GDL is a simple organic molecule which slowly hydrolysis in solution to form gluconic acid. This hydrolysis results in a gradual reduction in solution pH until a point at which carbonic acid may be formed from the divalent cation carbonate, such as CaCO₃ or BaCO₃, releasing active ions to induce alginate gelation. The rate of GDL hydrolysis, and therefore onset of gelation, may be altered by e.g. changing the temperature of the solution. As gelation proceeds at the activated crosslinker - alginate contact surface, there will be a reduction in the volume of the crosslinked alginate macromolecules which in turn reduces the effective concentration in the remainder of the alginate solution. This process continues during gelation and, as a result, a heterogenous hydrogel is produced, with a higher concentration of alginate at the activated crosslinker - alginate contact surface.

Another example of an activator composition is described in WO2013/076232 which is hereby incorporated by reference in its entirety. The activation system disclosed in said document involves the use of a hydrolase and a substrate being hydrolysable by the hydrolase. If the substrate which is hydrolysable by the hydrolase is present together with the inactive crosslinker, the hydrolase may be considered as the activator composition. If the hydrolase is present together with the inactive crosslinker, the substrate which is hydrolysable by the hydrolase may be considered to constitute the activator composition. If neither the hydrolase nor the substrate which is hydrolysable by the hydrolase is present together with the inactive crosslinker, it will be the hydrolase and the substrate which is hydrolysable by the hydrolase which is considered to constitute the activator composition. Thus, the activator composition may activate the inactive crosslinker directly or initiate a series of events resulting in the activation of the inactive crosslinker.

The term "hydrolase" as used herein is meant to encompass a hydrolase enabling the production of H₃O⁺ when mixing a solution comprising substrate(s) with another solution comprising the hydrolase. According to one embodiment of the invention, the hydrolase is a lipase. According to yet another embodiment of the present invention, the lipase is an acylhydrolase, more preferably a triacylglycerol lipase, such as for example the triacylglycerol lipase isolated from the yeast *Candida rugosa.* A suitable lipase is available from Sigma-Aldrich Co. LLC (L1754 - Type VII or L3001 Type I, CAS number 9001-62-1).

It is to be understood that any hydrolase resulting in the production of H₃O⁺ upon binding to its substrate may be used according to the present invention. An hydrolase that may be used may thus be selected from the group consisting of carboxylic ester hydrolases, oxaloacetase, glycosidases, ether hydrolases and hydrolases acting on carbon-nitrogen bonds other than peptide bonds in linear amides, such as chitin deacetylase.

Non-limiting examples of carboxylic ester hydrolases are carboxylesterase, triglycerol lipases, acetyl esterase, sterol esterase, L-arabinonolactonase, gluconolactonase, acylglycerol lipase, g-acetylglucose deacetylase, lipoprotein lipase, fatty acyl ethyl ester synthase, poly(3-hydroxybutyrate)depolymerase, and diacylglycerol acylhydrolase. Non-limiting examples of oxaloacetase are fumarylacetoacetase, acylpyruvate hydrolase, and acetylpyruvate hydrolase. A non-limiting example of a glycosidase is a-glucuronidase. A non-limiting example of an ether hydrolase is isochorismatase.

The substrate used in the forming of a matrix according to the present invention is a substrate which upon binding to the enzyme results in the production of H₃O⁺. The substrate may thus vary depending on the type of hydrolase used according to the present invention.

Suitable substrates according to the present invention are esters of organic acids, such as carboxylic acids.

According to one embodiment of the present invention, the substrate is a compound of formula I: 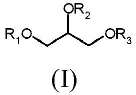 wherein R₁, R₂, and R₃ independently are the same or different and represents a straight or branched, substituted or non-substituted C₁-C₁₂ alkyl carbonyl chain, such as e.g. methanone, ethanone, acetone, butanone, pentanone, hexanone, heptanone, octanone, nonanone, decanone, dodecanone etc. According to one embodiment, R₁, R₂, and R₃ are each methanone. According to another embodiment, R₁, R₂, and R₃ are each ethanone. According to yet another embodiment, R₁, R₂, and R₃ is acetone. Substrates of the formula I is in particular useful when forming matrixes using a triacylglycerol lipase as the hydrolase according to the present invention. Upon binding to the, said ester of formula I are split into glycerol and a carboxylic acid, i.e. thus providing H₃O⁺.

The alkyl carbonyl chain may be branched or unbranced. The alkyl carbonyl chain may furthermore be substituted or unsubstituted. The skilled person will acknowledge, based on the teaching herein, that various substrate covered by the formula I may be used and may based on the teaching herein select the proper substrate to be used according to the present invention. The skilled person will thus acknowledge that the alkyl chain length may vary without affecting the ability of the enzyme to produce glycerol and a carboxylic acid of the substrate, thus resulting in the release of H₃O⁺ ions.

According to a preferred embodiment of the present invention, the substrate is selected from the group triacetin, tripropionin and tributyrin, of the formulas:

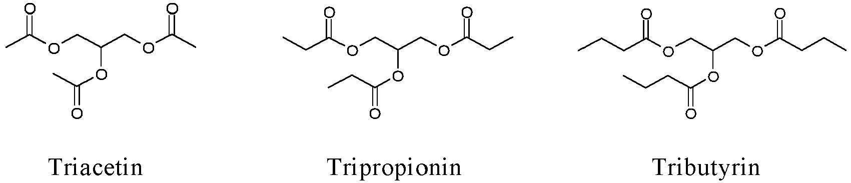

Thus, according to one embodiment, R₁, R₂, and R₃ represent C1-C4 alkyl carbonyl.

According to yet another embodiment of the present invention, the substrate present is selected from the group consisting of tripropionin and tributyrin.

According to the present invention, the mixing of the hydrolase and the substrate defined above results in the production of H₃O⁺. Said H₃O⁺ furthermore result in the release of ions from the inactive crosslinker.

Even though the hydrolase is not directly activating the inactive crosslinker, it is still considered to represent an activator composition as the hydrolase initiates a series of events resulting in the activation of the inactive crosslinker.

Thus, in one embodiment according to the present invention, the activator composition directly activates the inactive crosslinker. Examples of such activator compositions are acids, such as hydrochloric acid.

In a preferred embodiment according to the present invention, the activator composition indirectly activates the inactive crosslinker by initiating a series of events resulting in the activation of the inactive crosslinker. One example of such activator composition is a hydrolase which hydrolyzes a substrate resulting in the formation of an acid which in turn interacts with CaCO₃ resulting in the release of free calcium ions from said carbonate salt. Another example of an activator composition which indirectly activates the inactive crosslinker is GDL which needs to be hydrolyzed into the acid form before being able to activate the inactive crosslinker.

When preparing the sustained release composition of the present invention, it is preferred to use an activator composition which indirectly activates the inactive crosslinker. By using an activator composition which indirectly activates the inactive crosslinker, the rate of activation may be controlled thereby ensuring that gelation does not occur too fast.

In one embodiment according to the present invention, the inactive crosslinker and/or the activator composition are/is in solid form.

In one embodiment according to the present invention, the activator composition either directly activates the inactive crosslinker or initiates a series of events resulting in the activation of the inactive crosslinker.

In one embodiment according to the present invention, the activator composition comprises a hydrolase and the pre-mixture further comprises a substrate being hydrolysable by the hydrolase; or the premixture further comprises a hydrolase and the activator composition comprises a substrate being hydrolysable by the hydrolase.

In one preferred embodiment according to the present invention,
- the material to be released is non-human spermatozoa;
- the inactive crosslinker is a divalent cation carbonate, such as CaCO₃, BaCO₃ or any mixture thereof;
- the inactive crosslinker is in solid form; and
- the activator composition comprises a hydrolase and the pre-mixture further comprises a substrate being hydrolysable by the hydrolase; or
the premixture further comprises a hydrolase and the activator composition comprises a substrate being hydrolysable by the hydrolase; or
the activator composition comprises GDL.

A **third aspect** of the present invention relates to a sustained release composition, preferably the sustained release composition according to the first aspect of the present invention, being obtainable by the method according to the second aspect of the present invention.

A **fourth aspect** of the present invention relates to a container, such as an insemination straw or insemination tube, comprising the sustained release composition according to the first aspect of the present invention or the sustained release composition according to the third aspect of the present invention; wherein the material to be released preferably is non-human spermatozoa.

In one embodiment according to the present invention, the container is an insemination straw (1) comprising a tube extending between a first end (2) and a second end (3) and comprising a gas-permeable, liquid-tight plug (4), said plug being arranged in the tube in the vicinity of the first end of same and extending between a first end turned towards the first end of the tube (2) and a second end turned towards the second end of the tube (3).

In a second embodiment according to the present invention, the container is an insemination tube (1) comprising a tube extending between a first end (2) and a second end (3). An insemination tube suitable for insemination of animals like pigs does not have a gas-permeable liquid-tight plug as referred to in respect of insemination straws suitable for insemination of animals like cattle.

The sustained release composition according to the first aspect of the present invention or the sustained release composition according to the third aspect of the present invention, wherein the material to be released is non-human spermatozoa; may also be used in the breeding of animals.

Having generally described this invention, a further understanding can be obtained by reference to certain specific examples, which are provided herein for purposes of illustration only, and are not intended to be limiting unless otherwise specified.

### Examples

### Example 1: Preparation of bovine spermatozoa in alginate gels with varying mechanical strength within insemination straws

### Materials

The following chemicals were used: trizma hydrochloride, EDTA, D-(+)-Gluconic acid δ-lactone, NaHCO3, NaCl, glycerol (>99 %), fructose and sodium citrate from Sigma-Aldrich (St. Louis, USA). Glucose anhydrate from Apro (Oslo, Norway). ViCality AlbaFil and ViCality Ultra Heavy calcium carbonate from Brenntag Specialties (South Plainfield, USA) and sodium alginate from NovaMatrix, FMC BioPolymer AS (Sandvika, Norway).

### Source of spermatozoa

Bovine spermatozoa were collected at the Geno facilities at Hallsteingard in Trondheim and Store Ree in Stange, Norway.

### Buffer solutions

The following extender solutions were used:
- Extender for first dilution of spermatozoa: 1.45 g l⁻¹ Trizma hydrochloride glucose, 0.4 g l⁻¹ sodium citrate, 1 g l⁻¹ fructose, and 200 ml l⁻¹ egg yolk. The pH of the solution was adjusted to 6.4 by addition of NaOH.
- Extender solution for secondary dilution of spermatozoa: 1 g l⁻¹ ViCality Albafil calcium carbonate, 54 g l⁻¹ fructose, 170 g l⁻¹ glycerol and 10 g l⁻¹ sodium alginate. Both extenders contain standard antibiotic cocktail giving at least the final concentration required in EU dir 88/407.

### Preparation of the sustained release composition comprising spermatozoa

Bovine spermatozoa were harvested at the Geno facilities. Immediately after harvesting, the spermatozoa were diluted to a concentration of 133 x 10⁶ cells per ml in the extender solution for first time dilution. The resulting solution containing spermatozoa was then cooled to 4 °C. After cooling to 4 °C, the solution was mixed with an equal volume of the extender solution for secondary dilution.

Insemination straws were filled with 5 mg D-(+)-Gluconic acid δ-lactone or 5 mg of a mixture of D-(+)-Gluconic acid δ-lactone and ViCality Ultra heavy CaCO3 in a ratio of 1:1. The powder containing D-(+)-Gluconic acid δ-lactone or D-(+)-Gluconic acid δ-lactone and was positioned on the liquid side of the sealing plug in the insemination straws. The solution containing spermatozoa were then transferred to insemination straws and equilibrated at 4 °C for approximately 6 hours. The insemination straws were transferred to a N₂-freezer and frozen according to standard procedures for bull semen after the equilibration period at 4 °C.

### Example 2: Assessment of motility, mechanical strength and pH

Frozen semen straws, prepared according to the procedure described in example 1, were thawed by holding the straws in water bath at 37 °C for 30 seconds. The gel inside thawed insemination straws was sectioned into four equal parts, and the mechanical strength, pH and the motility of the spermatozoa were examined in each of the four sections.

The motility of the spermatozoa was assessed using microscopic evaluation. Prior to measurement of motility, the alginate gel was liquefied in modified IVT solution (3 g l⁻¹ glucose, 20 g l⁻¹ sodium citrate, 2.1 g l⁻¹ NaHCO3, 1.16 g l⁻¹ NaCl, 3 g l⁻¹ EDTA, pH 7.35) by adding the content of an insemination straw to 0.9 ml of modified IVT solution in an Eppendorf tube and shaking the tube carefully on a tube-tumbler for approximately 10 minutes. The tubes were preheated for minimum 15 minutes in a heat-block at 37 °C prior to assessment of motility. Approximately 3 µl of the solution was added to a preheated microscope slide and immediately inspected using a light microscope. The number of motile spermatozoa in each sample was estimated to the nearest 5% interval.

Approximately 50 - 60 % of the spermatozoa were motile in all 4 sections of the gel formed within the insemination straws. It was however observed significant differences in mechanical strength in the four sections. The section placed farthermost from the position where the CaCO3/D-(+)-Gluconic acid δ-lactone powder was positioned within the straws was in a nearly liquid state, while a rigid gel was observed in the section positioned close to where the powder was positioned. A varying mechanical strength was observed in the middle section of the straws, with the weakest mechanical strength being observed in the section farthermost from where the powder was positioned. The pH was measured in all 4 sections and the pH varied from 7.4 in the sections located farthest away from the where the powder was positioned in the straw to pH 6.8 in the section located closest to where the powder was positioned within the insemination straws.

### Example 3: Assessment of sperm quality of different part of the alginate gel

Frozen semen straws, prepared according to the procedure described in example 1 with modifications as outlined in table 1, were thawed by holding the straws in water bath at 37 °C for 30 seconds. The gel inside the thawed insemination straws was sectioned into four equal parts and the section farthermost from and the section next to the position where the CaCO3/D-(+)-Gluconic acid δ-lactone powder was positioned within the straws, were analyzed for viability and acrosome intactness by flow cytometry (BD AccuriTM C6 cytometer, Franklin Lakes, New Jersey, USA) and motility and progressive motility by CASA (Sperm Class Analyzer, SCA, Microptic, S.L.; Barcelona, Spain). Two gel parts with the same position within straw from each experiment were mixed in 200 µl dilution buffer (4.27 1 g l⁻¹ NaCL, 4.77 1 g l⁻¹ HEPES (MW 238.3), 5.21 1 g l⁻¹ HEPES Salt (MW 260.29), 3.0 1 g l⁻¹ glucose, 10 1 g l⁻¹ sodium citrate, 1.5 1 g l⁻¹ EDTA, pH 7.4, osm 313) in an Eppendorf tube. The tube was carefully mixed for 15 min at 37°C before flow cytometry and CASA analysis. The CASA analysis was done both in dilution buffer s above and after centrifugation at 800xg for 5 min and resolution of the pellet in the same amount of pre-warmed TALP buffer (20 mM HEPES, 20 mM HEPES salt, 87 mM NaCl, 3.1 mM KCl, 0.4 mM MgCl₂, 0.3 mM HaH₂PO₄, 2 mM CaCl₂, 1 mM NaPyr, 10 mM NaHCO₃, 21.6 mM NaLactate, 5 mM glucose, 50 µg/ml gentamycin, 6 mg/ml BSA, osm 292 pH 7.4).

### Assessment of motility and progressive motility by CASA

Each semen sample was tilted gently five times before loading an aliquot of 3 µl into a prewarmed Leja slide (Leja BV, Nieuw Vennep, The Netherlands). Each semen samples were analyzed in parallels on SCA (Sperm Class Analyzer, Microptic, Barcelona, Spain). The phase-contrast microscope were supplied with a prewarmed stage at 38°C and at 100× magnification. The number of analyzed spermatozoa per parallel were at least 600 sperm cells. Sperm cells were identified by head area of 30-70 µm2. Total motility was defined as percentage of sperm cells with mean VCL>15 µm/s. Progressivity was defined as above 70 % STR.

Initial percent mean (SD) motility and progressive motility were 45(7) and 36(6) for sperm cells from the section farthermost from the powder, and 51(16) and 39(15) respectively for the sperm cells from the section nearest to the end where the powder was positioned in the straw. In TALP buffer, the tendency was the same.

### Assessment of viability and acrosome intactness by flow cytometry (AccuriC6)

The samples were stained with the propidium iodid (PI, P4864, Sigma-Aldrich) for dead sperm cells and Lectin PNA from *Arachis hypogaea* (peanut) conjugated with Alexa Fluor® 488 (PNA-Alexa 488, L21409, Invitrogen) for acrosome reacted sperm cells. In addition, spermatozoa were stained with Mitotracker Orange (MO, M7510, Invitrogen). Briefly, sperm cells were diluted in PBS to a concentration of 1-2 million spermatozoa per ml (38°C) with fluorescent probes giving a final concentration of 0.15 µM MO, 7.5 µM PI and 0.05 µg/ml PNA-Alexa 488. The sperm samples were incubated for 15 minutes at room temperature before flow cytometry analysis. Sperm cell analyses were performed using a flow cytometer (BD AccuriTM C6 Cytometer, Franklin Lakes, New Jersey, USA) equipped with a blue (488 nm) and red laser (633 nm), two light scatter detectors, and four fluorescence detectors with optical filters (FL1-4: 533/30, 585/40, 670LP, 675/25). Validation beads (Spherotech 8-peaks validation beads (FL1-FL3) and Spherotech 6-peaks validation beads (FL4), BD Biosciences, Franklin Lakes, New Jersey, USA) for all four fluorescence channels FL1-4 were run daily. MO (excited by the 488 laser) in FL2 combined with PI (excited by the 488 laser) in FL3 (670LP) identified sperm cells. PNA-Alexa 488 was excited by the 488 laser and detected in FL1 (533/30). Compensation control was performed by running unstained and single stained samples. For each sample at least 6000 individual sperms were evaluated at flow rate of 300 to 500 sperms/s. Flow cytometry data was analysed using BD Accuri C6 software.

Percent mean (SD) acrosome intact live (AIL) sperm cells was 37(6) for sperm cells from the section farthermost from the powder, and 37(4) for the sperm cells from the section nearest to the end where the powder was positioned in the straw.

| Table 1 | | | | **AIL** | | **MOT** | | **Prog** | | **MotTALP** | | **ProgTALP** | |
|---|---|---|---|---|---|---|---|---|---|---|---|---|---|
| **Exp** | **Powder** | **Type CaCO₃** | **mM CaCO₃F₂** | **Part 1** | **Part 2** | **Part 1** | **Part 2** | **Part 1** | **Part 2** | **Part 1** | **Part 2** | **Part 1** | **Part 2** |
| 1 | CaCO₃:GDL (1:1) | Medium | 10 | 33 | 32 | 40.83 | 49.92 | 31.12 | 36.17 | 32.98 | 35.05 | 23.51 | 22.54 |
| 2 | GDL | Medium | 10 | 43 | 37 | 54.28 | 31.22 | 44.57 | 18.95 | 43.38 | 30.07 | 33.74 | 18.54 |
| 3 | CaCO₃:GDL (1:1) | Extra Heavy | 10 | 36 | 38 | 37.52 | 63.91 | 29.96 | 51.55 | 34.42 | 65.05 | 27.15 | 50.94 |
| 4 | CaCO₃:GDL (1:1) | Extra Heavy | 20 | 44 | 42 | 50.73 | 41.12 | 39.12 | 32.23 | 47.37 | 38.33 | 34.55 | 30.33 |
| 5 | CaCO₃:GDL (2:1) | Extra Heavy | 10 | 31 | 35 | 42.06 | 70.06 | 33.2 | 57.57 | 42.53 | 61.79 | 33.72 | 45.52 |
| | | | | | | | | | | | | | |
| **Mean** | | | | 37.4 | 36.8 | 45.08 | 51.24 | 35.59 | 39.29 | 40.13 | 46.05 | 30.53 | 33.57 |
| **SD** | | | | 5.9 | 3.7 | 7.1 | 16.0 | 6.1 | 15.5 | 6.2 | 16.2 | 4.9 | 14.2 |

Semen straws with varying mechanical strength obtained by different combinations of a total of 2 mg powder and extender (mM CaCO₃) were prepared as described (F₂ refers to extender solution for secondary dilution of spermatozoa) . Acrosome intact live (AIL) sperm cells was measured by flow cytometry, motility (Mot) and progressive motility (Prog) by CASA for the section farthermost from the position where the CaCO₃/D-(+)-Gluconic acid δ-lactone powder (Part 1) and the section closest to where the powder was positioned (Part 2). Sperm cell samples were centrifuged and resolved in TALP buffer and motility (MotTALP) and progressivity (ProgTALP) measured by CASA. The ratio referred to in the column entitled "powder" refers to molar ratio. The type of CaCO₃ referred to above is ViCality medium and ViCality Extra Heavy respectively.

### Example 4: Assessment of dissolution rate

Frozen semen straws, prepared according to the procedure described in example 1 with modifications as outlined in table 2, were thawed by holding the straws in water bath at 37°C for 30 seconds. The gel inside thawed insemination straws was sectioned into three equal parts and the section farthermost from and the section next to the position where the CaCO3/D-(+)-Gluconic acid δ-lactone powder was positioned within the straws, were analyzed for *in vitro* dissolution rate.

### In-vitro assessment of dissolution rate in the absence of calcium

3 cm of the semen straw from each end were transferred to 3 ml of pre-warmed buffer (133 mM NaCl, 3.5 mM KCl, 1.5 mM MgSO4x7HO, 4 mMKH2PO4, 3.5 mM glucose, 20 mM HEPES, 0.1 mM NaPyr, 1.1 mM NaLactat, 1 µg/ml gentamycin) in 15 ml Falcon tubes and incubated at 38°C with very gentle tilting (PMR-30, Grant, England) for observation of dissolution rate. The gel first becomes thinner, and thereafter dissolves in several parts. When all the gel from the insemination straw was dissolved in small particles, and all parts were less than 1 mm in size, the gel was considered as resolved. Two samples from each treatment were analyzed. As shown in table 2, the gel positioned farthermost from the powder was dissolved within 8 minutes while the gel nearest to the powder was dissolved after 8X to 18X longer time (59-140 minutes), conforming longer dissolution rate.

### In-vitro assessment of dissolution rate in the presence of calcium

3 cm of the semen straw from each end were transferred to 3 ml of pre-warmed buffer (0.2 mM CaCl₂, 133 mM NaCl, 3.5 mMKCl, 1.5 mM MgSO4x7HO, 4 mMKH2PO4, 3.5 mM glucose, 20 mM HEPES, 0.1 mM NaPyr, 1.1 mM NaLactat, 1 µg/ml gentamycin) in 15 ml Falcon tubes and incubated at 38 °C with very gentle tilting (PMR-30, Grant, England) for observation of dissolution rate. After one-hour part 1 consisted of several small pieces, while part 2 still was a gel. After three hours, part 1 was dissolved while part 2 was divided in pieces. Part 2 became more dissolved during time, but was still not completely dissolved after 24 hours.

The dissolution rate *in vitro* is dependent on the concentration of calcium ions. By increasing the calcium concentration in the buffer, the dissolution time of the gel also increases, but differences between gels with different gel strength remain the same.

Ions, including calcium and magnesium, are known to play an important role in the formation of oviduct and uterine fluid (vet. 2012 Spring; 3(2): 137-141). They move through the epithelial cells of the uterus into the lumen of the reproductive tract causing a concentration gradient which in turn causes an osmotic gradient providing the driving force to transport water by osmosis out of the epithelial cells into the uterine lumen. Thus, it seems clear that the presence of calcium ions in the reproductive tract will affect the dissolution rate of calcium alginate in an in-vivo situation. In order to investigate this further, experiments have been conducted to study calcium alginate dissolution rate in a close to in-vivo environment.

### "In-vivo like" assessment of dissolution rate

The resolution of the gel was also assessed under more *in vivo* like conditions. Reproduction organ from cow (close to ovulation time) was collected at the slaughter house. The uterus was opened by scissors, and semen straw prepared as described above was positioned in the uterus as a string. The uterus was closed and incubated overnight at room temperature. After 20 hours, part 1 of the gel was almost totally dissolved while part 2 was still a firm and clear gel.

**Table 2:**

| | | | **Mot** | | **AIL** | | **Dissolution rate (absence of Ca)** | |
|---|---|---|---|---|---|---|---|---|
| **Exp** | **Extender** | **mM CaCO₃ F₂** | **Part 1** | **Part 2** | **Part 1** | **Part 2** | **Part 1** | **Part 2** |
| 1 | Control (Biladyl®) | - | 58% | 58% | 40% | 58% | 1X | 1X |
| 2 | Ultra Heavy CaCO3 :GDL 1:1 | 10 | 56% | 52% | 56% | 42% | 1X | 8X |
| 3 | Ultra Heavy CaCO3:GDL 1:1 | 20 | 56% | 52% | 62% | 52% | 1X | 11X |
| 4 | Ultra Heavy CaCO3:GDL 1:1 | 10 | 55% | 52% | 39% | 43% | 1X | 18X |
| 5 | Ultra Heavy CaCO3:GDL 1:1 | 20 | 57% | 50% | 53% | 42% | 1X | 19X |

Semen straws with varying mechanical strength obtained by different combinations of powder and extender (mM CaCO₃) were prepared as described with modifications as outlined in table 2. Motility (Mot) was observed objectively and acrosome intact live (AIL) was measured by flow cytometry for the sections farthermost from the position where the CaCO₃/D-(+)-Gluconic acid δ-lactone powder (Part 1) and section close to where the powder was positioned (Part 2). Dissolution rate was followed and given relative to Part 1. The ratio referred to in the column entitled "extender" refers to molar ratio. The type of CaCO₃ referred to in the column entitled "extender" is ViCality Ultra Heavy.

### Example 5: Determination of mechanical strength of different alginate gels with immobilized sperm cells

### Materials

The following chemicals were used: trizma hydrochloride, EDTA, D-(+)-Gluconic acid δ-lactone, NaHCO3, NaCl, glycerol (>99 %), fructose and sodium citrate from Sigma-Aldrich (St. Louis, USA). Glucose anhydrate from Apro (Oslo, Norway). Eskal500 calcium carbonate from KSL staubtechnik gmbh (Lauingen, Germany) and sodium alginate (UP LVG and UP VLVG) from NovaMatrix, FMC BioPolymer AS (Sandvika, Norway). Standard insemination straw 0.25 ml (French mini straws (IMV, L'Aigle, France)).

### Source of spermatozoa

Bovine spermatozoa were collected at the Geno facilities at Hallsteingård in Trondheim and Store Ree in Stange, Norway.

### Buffer solutions

The following extender solutions were used:
Extender for first dilution of spermatozoa: 1.45 g l⁻¹ Trizma hydrochloride glucose, 0.4 g l⁻¹ sodium citrate, 1 g l⁻¹ fructose, and 200 ml l⁻¹ egg yolk. The pH of the solution was adjusted to 6.4 by addition of NaOH.

Extender solution for secondary dilution of spermatozoa: 0.5 g l⁻¹ Eskal500 calcium carbonate, 54 g l⁻¹ fructose, 170 g l⁻¹ glycerol and 12 g l⁻¹ sodium alginate (mixture of UP LVG and UP VLVG). Both extenders contain standard antibiotic cocktail giving at least the final concentration required in EU dir 88/407.

### Dilution, immobilization and cryoconservation of bull spermatozoa

Bovine spermatozoa were harvested at the Geno facilities. Immediately after harvesting, the spermatozoa were diluted to a concentration of 133 x 10⁶ cells per ml in the extender solution for first time dilution. The resulting solution containing spermatozoa was then cooled to 4 °C. After cooling to 4 °C, the solution was mixed with an equal volume of the extender solution for secondary dilution. The mixture was added D-(+)-Gluconic acid δ-lactone to a final concentration of 55 mM in order to initialize gelling, and filled on semen straws. The straws were stored at 4 °C for approximately 4.5 hours and frozen in liquid N₂ according to standard procedures for cryoconservation. The ratio of UP LVG and UP VLVG in the solution for secondary dilution was varied in order to create alginate gels with varying mechanical strength.

### Evaluation of mechanical strength

10 straws of frozen semen per processing were thawed at 37°C for 1 minute and subsequently aligned in parallel in close contact and centered on a metal plate. A Texture analyzer TA XT Plus (Stable micro systems, Godalming, Surrey, UK) equipped with a P/35 probe (35 mm DIA CYLINDER ALUMINIUM) was used in order to quantify the mechanical strength of the gel from the semen straws. The measurements (test mode compression) was run using a 1 kg load cell at a test speed 0.1 mm/s to 80 % strain. The mechanical strength was quantified as the initial linear incline (between 0.05 and 0.1 mm) of the force against deformation of the gel. The mechanical strength of gels from straws characterized as weak or soft by manual assessments as in example 1 was measured to 43 g/mm. The mechanical strength of gels from straws characterized as strong by manual assessments was measured to 90 g/mm.

## Claims

1. A sustained release composition comprising a material to be released embedded in a matrix, the matrix comprising an ionically crosslinked polymer and having at least a first and a second section with distinct mechanical strength; wherein the ionically crosslinked polymer is ionically crosslinked alginate, the alginate having more guluronic acid residues than mannuronic acid residues.

2. The sustained release composition according to claim 1, wherein the ionically crosslinked alginate is divalent cation crosslinked alginate.

3. The sustained release composition according to claim 1 or claim 2, wherein the material to be released is non-human spermatozoa.

4. The sustained release composition according to claim 1, wherein
the first section has a mechanical strength that is at least 10% higher, such as at least 20% higher, than the mechanical strength of the second section; or
the second section has a mechanical strength in the range 10 to 190 g/mm and the first section has a mechanical strength that is at least 10% higher, such as at least 20% higher, than the mechanical strength of the second section; or
the second section has a mechanical strength in the range 10 to 190 g/mm and the first section has a mechanical strength in the range 10 to 190 g/mm, with the proviso that the first and a second sections have distinct mechanical strength; or the mechanical strength of the first section : the mechanical strength of the second section ratio is at least 1.1, such as at least 1.2.

5. Method for preparing the sustained release composition of claim 1, the method comprising the following steps:
- mixing the material to be released with a composition comprising ionically crosslinkable alginate thereby forming a pre-mixture;
- bringing the pre-mixture into contact with an inactive crosslinker and an activator composition; and
- allowing the ions and the ionically crosslinkable alginate to form ionically crosslinked alginate and thereby obtain the sustained release composition of claim 1;
wherein
the ionically crosslinkable alginate has more guluronic acid residues than mannuronic acid residues;
the inactive crosslinker is capable of releasing ions upon activation, the ions being suitable for crosslinking the ionically crosslinkable alginate; and
the activator composition comprising one or more compounds capable of activating the inactive crosslinker.

6. The method according to claim 5, wherein the material to be released is non-human spermatozoa.

7. The method of claim 5, wherein the inactive crosslinker is a divalent cation carbonate, such as CaCO₃, BaCO₃ or any mixture thereof.

8. The method of claim 5, wherein the activator composition either directly activates the inactive crosslinker or initiates a series of events resulting in the activation of the inactive crosslinker.

9. The method of claim 5, wherein
- the pre-mixture is brought into contact with the inactive crosslinker and then subsequently the activator composition is added; or
- the pre-mixture is brought into contact with the activator composition and then subsequently the inactive crosslinker is added; or
- the pre-mixture is brought into contact with the activator composition and the inactive crosslinker, the activator composition and the inactive crosslinker being simultaneously added to the pre-mixture.

10. The method of claim 5, wherein the activator composition comprises glucono-δ-lactone.

11. The method of claim 5, wherein the activator composition comprises a hydrolase and the pre-mixture further comprises a substrate being hydrolysable by the hydrolase; or the premixture further comprises a hydrolase and the activator composition comprises a substrate being hydrolysable by the hydrolase.

12. The method of claim 5, wherein
- the material to be released is non-human spermatozoa;
- the inactive crosslinker is a divalent cation carbonate, such as CaCO₃, BaCO₃ or any mixture thereof;
- the inactive crosslinker is in solid form; and
- the activator composition comprises a hydrolase and the pre-mixture further comprises a substrate being hydrolysable by the hydrolase; or
the premixture further comprises a hydrolase and the activator composition comprises a substrate being hydrolysable by the hydrolase; or
the activator composition comprises glucono-δ-lactone.

13. A sustained release composition being obtainable by the method according to claim 5.

14. A container comprising the sustained release composition according to claim 1 or the sustained release composition according to claim 13; wherein the material to be released is non-human spermatozoa.

## Patentansprüche

1. Zusammensetzung mit anhaltender Freisetzung, die ein freizusetzendes Material eingebettet in eine Matrix umfasst, wobei die Matrix ein ionisch vernetztes Polymer umfasst und wenigstens einen ersten und einen zweiten Bereich mit unterschiedlicher mechanischer Festigkeit aufweist; wobei das ionisch vernetzte Polymer ionisch vernetztes Alginat ist, wobei das Alginat mehr Guluronsäurereste als Mannuronsäurereste hat.

2. Zusammensetzung mit anhaltender Freisetzung nach Anspruch 1, wobei das ionisch vernetzte Alginat ein mit zweiwertigem Kation vernetztes Alginat ist.

3. Zusammensetzung mit anhaltender Freisetzung nach Anspruch 1 oder Anspruch 2, wobei das freizusetzende Material nicht-menschliche Spermatozoen ist.

4. Zusammensetzung mit anhaltender Freisetzung nach Anspruch 1, wobei
der erste Bereich eine mechanische Festigkeit hat, die wenigstens 10% höher wie z.B. wenigstens 20% höher ist als die mechanische Festigkeit des zweiten Bereichs; oder
der zweite Bereich eine mechanische Festigkeit im Bereich von 10 bis 190 g/mm hat und der erste Bereich eine mechanische Festigkeit hat, die wenigstens 10% höher wie z.B. wenigstens 20% höher ist als die mechanische Festigkeit des zweiten Bereichs; oder
der zweite Bereich eine mechanische Festigkeit im Bereich von 10 bis 190 g/mm hat und der erste Bereich eine mechanische Festigkeit von 10 bis 190 g/mm hat, mit der Maßgabe, dass der erste und ein zweiter Bereich unterschiedliche mechanische Festigkeit haben; oder
das Verhältnis mechanische Festigkeit des ersten Bereichs : mechanische Festigkeit des zweiten Bereichs wenigstens 1,1 wie z.B. wenigstens 1,2 ist.

5. Verfahren zur Herstellung der Zusammensetzung mit anhaltender Freisetzung nach Anspruch 1, wobei das Verfahren die folgenden Schritte umfasst:
- Mischen des freizusetzenden Materials mit einer Zusammensetzung, die ionisch vernetzbares Alginat umfasst, wodurch eine Vormischung gebildet wird;
- In-Kontakt-Bringen der Vormischung mit einem inaktiven Vernetzer und einer Aktivatorzusammensetzung und
- Ermöglichen, dass die Ionen und das ionisch vernetzbare Alginat ionisch vernetztes Alginat bilden und dadurch die Zusammensetzung mit anhaltender Freisetzung nach Anspruch 1 erhalten wird;
wobei
das ionisch vernetzbare Alginat mehr Guluronsäurereste als Mannuronsäurereste hat;
der inaktive Vernetzer in der Lage ist, bei Aktivierung Ionen freizusetzen, wobei die Ionen zur Vernetzung des ionisch vernetzbaren Alginats geeignet sind; und
die Aktivator-Zusammensetzung eine oder mehrere Verbindung/en umfasst, die in der Lage ist/sind, den inaktiven Vernetzer zu aktivieren.

6. Verfahren nach Anspruch 5, wobei das freizusetzende Material nicht-menschliche Spermatozoen ist.

7. Verfahren nach Anspruch 5, wobei der inaktive Vernetzer ein zweiwertiges Kation-Carbonat, wie z.B. CaCO₃, BaCO₃ oder eine beliebige Mischung davon, ist

8. Verfahren nach Anspruch 5, wobei die Aktivator-Zusammensetzung den inaktiven Aktivator entweder direkt aktiviert oder eine Reihe von Ereignissen initiiert, die in der Aktivierung des inaktiven Vernetzers resultiert.

9. Verfahren nach Anspruch 5, wobei
- die Vormischung mit dem inaktiven Vernetzer in Kontakt gebracht wird und dann anschließend die Aktivator-Zusammensetzung zugegeben wird, oder
- die Vormischung mit der Aktivator-Zusammensetzung in Kontakt gebracht wird und dann anschließend der inaktive Vernetzer zugegeben wird, oder
- die Vormischung mit der Aktivator-Zusammensetzung und dem inaktiven Vernetzer in Kontakt gebracht wird, wobei die Aktivator-Zusammensetzung und der inaktive Vernetzer gleichzeitig zu der Vormischung gegeben werden.

10. Verfahren nach Anspruch 5, wobei die Aktivator-Zusammensetzung Glucono-δ-lacton umfasst.

11. Verfahren nach Anspruch 5, wobei die Aktivator-Zusammensetzung eine Hydrolase umfasst und die Vormischung ferner ein Substrat, das durch die Hydrolase hydrolysierbar ist, umfasst, oder die Vormischung ferner eine Hydrolase umfasst und die Aktivator-Zusammensetzung ein Substrat, das durch die Hydrolase hydrolysierbar ist, umfasst.

12. Verfahren nach Anspruch 5, wobei
- das freizusetzende Material nicht-menschliche Spermatozoen ist;
- der inaktive Vernetzer ein zweiwertiges Kation-Carbonat, wie z.B. CaCO₃, BaCO₃ oder eine beliebige Mischung davon, ist;
- der inaktive Vernetzer in fester Form ist und
- die Aktivator-Zusammensetzung eine Hydrolase umfasst und die Vormischung ferner ein Substrat, das durch die Hydrolase hydrolysierbar ist, umfasst; oder
- die Vormischung ferner eine Hydrolase umfasst und die Aktivator-Zusammensetzung ein Substrat, das durch die Hydrolase hydrolysierbar ist, umfasst; oder
- die Aktivator-Zusammensetzung Glucono-δ-lacton umfasst.

13. Zusammensetzung mit anhaltender Freisetzung, die durch das Verfahren nach Anspruch 5 erhältlich ist.

14. Behälter, der die Zusammensetzung mit anhaltender Freisetzung nach Anspruch 1 oder die Zusammensetzung mit anhaltender Freisetzung nach Anspruch 13 umfasst, wobei das freizusetzende Material nicht-menschliche Spermatozoen ist.

## Revendications

1. Composition à libération prolongée comprenant une matière à libérer incorporée dans une matrice, la matrice comprenant un polymère réticulé ioniquement et ayant au moins une première et une deuxième section avec une résistance mécanique distincte ; dans laquelle le polymère réticulé ioniquement est un alginate réticulé ioniquement, l'alginate ayant plus de résidus acide guluronique que de résidus acide mannuronique.

2. Composition à libération prolongée selon la revendication 1, dans laquelle l'alginate réticulé ioniquement est un alginate réticulé par cations divalents.

3. Composition à libération prolongée selon la revendication 1 ou la revendication 2, dans laquelle la matière à libérer est constituée de spermatozoïdes non humains.

4. Composition à libération prolongée selon la revendication 1, dans laquelle
la première section a une résistance mécanique qui est supérieure d'au moins 10 %, telle que supérieure d'au moins 20 %, à la résistance mécanique de la deuxième section ; ou
la deuxième section a une résistance mécanique dans la plage de 10 à 190 g/mm et la première section a une résistance mécanique qui est supérieure d'au moins 10 %, telle que supérieure d'au moins 20 %, à la résistance mécanique de la deuxième section ; ou
la deuxième section a une résistance mécanique dans la plage de 10 à 190 g/mm et la première section a une résistance mécanique dans la plage de 10 à 190 g/mm, à condition que les première et deuxième sections aient une résistance mécanique distincte ; ou
le rapport résistance mécanique de la première section : résistance mécanique de la deuxième section est d'au moins 1:1, tel que d'au moins 1:2.

5. Procédé de préparation de la composition à libération prolongée de la revendication 1, le procédé comprenant les étapes suivantes :
- le mélange de la matière à libérer avec une composition comprenant un alginate réticulable ioniquement pour ainsi former un pré-mélange ;
- la mise en contact du pré-mélange avec un agent de réticulation inactif et une composition d'activateur ; et
- le fait de laisser les ions et l'alginate réticulable ioniquement former un alginate réticulé ioniquement et ainsi obtenir la composition à libération prolongée de la revendication 1 ;
dans lequel
l'alginate réticulable ioniquement a plus de résidus acide guluronique que de résidus acide mannuronique ;
l'agent de réticulation inactif est capable de libérer des ions lors d'une activation, les ions étant appropriés pour réticuler l'alginate réticulable ioniquement ; et
la composition d'activateur comprenant un ou plusieurs composés capables d'activer l'agent de réticulation inactif.

6. Procédé selon la revendication 5, dans lequel la matière à libérer est constituée de spermatozoïdes non humains.

7. Procédé selon la revendication 5, dans lequel l'agent de réticulation inactif est un carbonate à cations divalents, tel que CaCO₃, BaCO₃ ou tout mélange de ceux-ci.

8. Procédé selon la revendication 5, dans lequel la composition d'activateur soit active directement l'agent de réticulation inactif, soit déclenche une série d'événements aboutissant à l'activation de l'agent de réticulation inactif.

9. Procédé selon la revendication 5, dans lequel
- le pré-mélange est mis en contact avec l'agent de réticulation inactif, puis, par la suite, la composition d'activateur est ajoutée ; ou
- le pré-mélange est mis en contact avec la composition d'activateur, puis, par la suite, l'agent de réticulation inactif est ajouté ; ou
- le pré-mélange est mis en contact avec la composition d'activateur et l'agent de réticulation inactif, la composition d'activateur et l'agent de réticulation inactif étant ajoutés simultanément au pré-mélange.

10. Procédé selon la revendication 5, dans lequel la composition d'activateur comprend de la glucono-δ-lactone.

11. Procédé selon la revendication 5, dans lequel la composition d'activateur comprend une hydrolase et le pré-mélange comprend en outre un substrat qui est hydrolysable par l'hydrolase ; ou le pré-mélange comprend en outre une hydrolase et la composition d'activateur comprend un substrat qui est hydrolysable par l'hydrolase.

12. Procédé selon la revendication 5, dans lequel
- la matière à libérer est constituée de spermatozoïdes non humains ;
- l'agent de réticulation inactif est un carbonate à cations divalents, tel que CaCO₃, BaCO₃ ou tout mélange de ceux-ci ;
- l'agent de réticulation inactif est sous forme solide ; et
- la composition d'activateur comprend une hydrolase et le pré-mélange comprend en outre un substrat qui est hydrolysable par l'hydrolase ; ou
le pré-mélange comprend en outre une hydrolase et la composition d'activateur comprend un substrat qui est hydrolysable par l'hydrolase ; ou
la composition d'activateur comprend de la glucono-δ-lactone.

13. Composition à libération prolongée qui peut être obtenue par le procédé selon la revendication 5.

14. Contenant comprenant la composition à libération prolongée selon la revendication 1 ou la composition à libération prolongée selon la revendication 13 ; dans lequel la matière à libérer est constituée de spermatozoïdes non humains.
